# EUROPÄISCHE PATENTSCHRIFT

(11) **EP 1 558 557 B1**
(45) Veröffentlichungstag und Bekanntmachung des Hinweises auf die Patenterteilung: **14.03.2007**
(21) Anmeldenummer: 04709624.3
(22) Anmeldetag: 10.02.2004
(51) Int. Cl.: C07C 67/035, C07C 69/63, C07C 29/09, C07C 31/04

(54) **VERFAHREN ZUR KATALYTISCHEN OXIDATION VON METHAN ZU METHANOL ÜBER METHANOLESTER**
METHOD FOR CATALYTIC OXIDATION OF METHANE TO FORM METHANANOL VIA METHANOL ESTERS
PROCEDE D'OXYDATION CATALYTIQUE DE METHANE EN METHANOL PAR L'INTERMEDIAIRE D'ESTER DE METHANOL

(30) Priorität: 10.02.2003 DE 10305377
(43) Veröffentlichungstag der Anmeldung: 03.08.2005
(73) Patentinhaber: SÜD-CHEMIE AG, 80333 München (DE)
(72) Erfinder: WANNINGER, Klaus, 83059 Kolbermoor (DE); REIMER, Alfred, 93437 Furth im Wald (DE); SCHMIDT, Friedrich, 83024 Rosenheim (DE); MALETZ, Gerd, 83043 Bad Aibling (DE); STRASSNER, Thomas, 90459 Nürnberg (DE); MÜHLHOFER, Michael, 80339 München (DE); HERMANN, Wolfgang, 85354 Freising (DE)
(74) Vertreter: Westendorp, Michael Oliver
(86) Internationale Anmeldenummer: PCT/EP2004/001215
(87) Internationale Veröffentlichungsnummer: WO 2004/069784

(56) Entgegenhaltungen:
- M.N. VARGAFTIK, ET AL.: "Highly selective partial oxidiation of methane to methyl trifluoroacetate" JOURNAL OF THE CHEMICAL SOCIETY, CHEMICAL COMMUNICATIONS., Nr. 15, 1. August 1990 (1990-08-01), Seiten 1049-1050, XP002282184 ROYAL SOCIETY OF CHEMISTRY, LETCHWORTH, GB in der Anmeldung erwähnt
- L.T. KANERVA: "Temperature dependence of activation parameters in the neutral ester hydrolysis in methanol-water solutions" ACTA CHEMICA SCANDINAVICA, SERIES B - ORGANIC CHEMISTRY AND BIOCHEMISTRY., Bd. 37, Nr. 9, 1983, Seiten 755-760, XP002282649 MUNKSGAARD, COPENHAGEN, DK

## Beschreibung

Die Erfindung betrifft ein Verfahren zur katalytischen Oxidation von Methan zu Methanol über Methanolester.

Die direkte Oxidation von Methan zu Methanol ist extrem schwierig, weil die Bindungsstärke der CH-Bindung der CH₃-Gruppe im Produkt Methanol geringer ist als im Methan, weshalb diese Bindung leichter angegriffen wird als in Methan. Eine hohe Selektivität ist also nahezu unmöglich. Es gibt daher einige Versuche, in Gegenwart starker Säuren die Ester des Methanols herzustellen. Diese sind dann stabiler gegen die Weiteroxidation.

Ein solches System ist mit Quecksilbersalzen (US-A-5,306,855) oder mit Platin-Komplexen (US-A5,233,113) als Katalysatoren in Schwefelsäure zum Methylsulfat gelungen. Nachteil dieser Systeme ist, dass nach erfolgter Hydrolyse der Methylsulfate zu Methanol und Schwefelsäure die Schwefelsäure nicht wieder einfach destillativ zur reinen Schwefelsäure aufkonzentriert werden kann und nur eine zu geringe Konzentration an Methylsulfat im Oleum erreicht wird. Eine einfache Abtrennung ist deshalb nicht möglich.

Andere Versuche erfolgten in Trifluoressigsäure, wobei deren Methylester erhalten wurden. Ein Katalysatorsystem dazu sind Palladium-Carbenkomplexe mit Peroxodisulfat als Oxidationsmittel (Deutsche Patentanmeldung 101 51 660.6). Nachteile dieses Systems sind die teuren Palladiumkomplexe und das teure Oxidationsmittel Peroxodisulfat.

Aus der Literaturstelle J. Chem. Soc. Chem. Commun. 1990 (1049 - 1050) (Verf. M.N. Vargaftik, I.P. Stolarov, und I.I. Moiseev) ist ein Verfahren zur hochselektiven partiellen Oxidation von Methan zu Methyltrifluoracetat bekannt. Nach diesem Verfahren wird Methan stöchiometrisch in Gegenwart von Kobalt(III)-trifluoracetat in CF₃CO₂H-Lösung bei 150 bis 180°C und 10 bis 40 bar Methandruck ohne Sauerstoff oxidiert, wobei Methyltrifluoracetat in einer Ausbeute von 90%, bezogen auf Co(III), erhalten wird, das hierbei zu Co(II) reduziert wird. In Gegenwart von Sauerstoff (1 bis 5 bar) wird die Reaktion katalytisch, wobei Methyltrifluoracetat erhalten wird. Laut Aussage des Artikels werden 400% Ausbeute erhalten, bezogen auf Co(III). Es wird erwähnt, dass die stöchiometrische Reaktion auch mit Mn(III)-trifluoracetat möglich ist, aber nur mit 30% Ausbeute.

Es geht aus dieser Literaturstelle nicht hervor, unter welchen Bedingungen die Versuche durchgeführt wurden, welche wirklichen Ausbeuten, bezogen auf Methan, erzielt wurden und wie die Umsätze berechnet wurden. Da keine Katalysatormengen angegeben sind, war eine Reproduktion nicht möglich. Außerdem wurde festgestellt, dass bei dieser Reaktion Kobaltfluorid ausfällt. Dies zeigt, dass Trifluoressigsäure zersetzt wird.

In der WO 92/17425 ist ein Verfahren zur selektiven Oxidation organischer Verbindungen bekannt, mit welchem beispielsweise Methanol aus Methan hergestellt werden kann. Als Oxidationsmittel wird Sauerstoff in Verbindung mit Cu(I)-Verbindungen verwendet. Aus der Cu(I)-Verbindung und Sauerstoff wird zunächst ein CH₃-Radikal gebildet, das mit Cu(II) und Trifluoressigsäure zum Trifluoressigsäuremethylester und einer Cu(I)-Verbindung umgesetzt wird. Für eine Anwendung des Verfahrens im industriellen Maßstab wird vorgeschlagen, das bei der Reaktion anfallende Cu(II) beispielsweise elektrochemisch wieder zu Cu(I) zu reduzieren. Die bei diesem Verfahren eingesetzten Kupferverbindungen wirken also nicht katalytisch.

Der Erfindung lag die Aufgabe zugrunde, unter Verwendung preisgünstigster Oxidationsmittel die Ausbeute an Methyltrifluoracetat zu erhöhen.

Es wurde überraschenderweise gefunden, dass die Zugabe von Anhydriden, wie z.B. Trifluoressigsäure, die Ausbeute erhöht. Außerdem wurde überraschenderweise gefunden, das der Zusatz nicht fluorierter Carbonsäuren die Ausfällung von Kobaltfluorid verhindert.

Gegenstand der Erfindung ist somit ein Verfahren zur Oxidation von Methan zu Methanol über Methanolester, wobei die Oxidation des Methans mit Sauerstoff oder einem sauerstoffhaltigen Gas in Gegenwart eines oder mehrerer Salze aus der Gruppe der Übergangsmetalle, die ein Redoxpaar mit einem Normalpotential > 0,6 Volt, vorzugsweise > 0,7 Volt, bilden, als Katalysator in Trifluoressigsäure durchgeführt und die Ester, vorzugsweise nach destillativer Abtrennung, anschließend zu Methanol hydrolysiert werden. Das Verfahren ist dadurch gekennzeichnet, dass man die Oxidation in einer Mischung aus Trifluoressigsäure und einem Carbonsäureanhydrid und/oder in einer Mischung aus Trifluoressigsäure und einer anderen, nicht-fluorierten Carbonsäure bei Temperaturen von mehr als 80°C durchführt. Vorzugsweise wird als Carbonsäureanhydrid Trifluoressigsäureanhydrid verwendet. Als Beispiel für die andere, nicht-fluorierte Carbonsäure kann z.B. Essigsäure genannt werden.

Die Hydrolyse des Esters kann in an sich bekannter Weise erfolgen. Aufgrund ihrer hydrophoben Eigenschaften bildet dabei Trifluoressigsäure kein Azeotrop mit Wasser und lässt sich nach der Hydrolyse des Trifluoressigsäuremethylesters destillativ zurückgewinnen.

Durch die Verwendung einer Mischung aus Trifluoressigsäure und Trifluoressigsäureanhydrid konnte die Ausbeute an Methylester unter sonst identischen Bedingungen deutlich gesteigert werden im Vergleich zur Reaktion in reiner Trifluoressigsäure. Verschiedene mögliche Ursachen kommen dafür in Betracht. Die Reaktion wird an sich unter wasserfreien Bedingungen durchgeführt. Es kann jedoch sein, dass Spuren von Wasser noch im Lösungsmittel Trifluoressigsäure enthalten sind bzw. beispielsweise durch die verwendeten Metallsalze eingeschleppt werden. Trifluoressigsäureanhydrid könnte diese Spuren von Wasser entfernen, welche die wahrscheinlich radikalische Reaktion stören. Es könnte auch sein, dass aus Sauerstoff und der protischen Säure in diesen Radikalreaktionen Wasser als Nebenprodukt gebildet wird, welches von dem Anhydrid entfernt wird. Über den Mechanismus, der dieser Ausbeutesteigerung zugrunde liegt, kann also nichts gesagt werden. Die Messungen des Umsatzes nach unterschiedlichen Zeitpunkten legen allerdings nahe, dass durch diese Maßnahme eine Stabilisierung des katalytischen Systems erfolgt, so dass mehr Reaktionen an einem aktiven Metallatom ablaufen.

In jedem Fall erlaubt die Verwendung von Trifluoressigsäureanhydrid auch die Verwendung von billigeren wasserhaltigen Metallsalzen als Katalysatoren. So konnten beliebige Kobaltsalze, z.B. auch das billigere wasserhaltige Kobaltacetat, als Katalysatorvorstufe eingesetzt werden im Vergleich zu dem sehr viel teureren Kobalttrifluoracetat, das Vargaftik et al. in ihrer Publikation verwenden.

Wie im Vergleichsbeispiel wird auch in dieser Reaktion mit der höheren Ausbeute ein Niederschlag von Kobaltfluorid beobachtet.

Das Gewichtsverhältnis zwischen Trifluoressigsäure und Carbonsäureanhydrid beträgt vorzugsweise 99:1 bis 1:99, insbesondere 99:1 bis 20:80 bzw. 70:30 bis 30:70.

Nach einer weiteren Ausgestaltung der Erfindung wird die Reaktion in einer Mischung aus Trifluoressigsäure und einer nicht fluorierten Carbonsäure durchgeführt. So konnten in einer Mischung aus Trifluoressigsäure und Essigsäure ebenfalls höhere Ausbeuten der beiden möglichen Ester der beiden Säuren gefunden werden. Außerdem wurde kein Kobaltfluoridniederschlag mehr gefunden.

Die Ursache für die bessere Stabilisierung des katalytischen Systems ist noch unklar.

Das Gewichtsverhältnis zwischen Trifluoressigsäure und nicht fluorierter Carbonsäure beträgt vorzugsweise 99:1 bis 1:99, besonders bevorzugt 99:1 bis 5:95 bzw. 99:1 bis 10:90, insbesondere 30:70 bis 70:30.

Als Katalysatorvorstufen kommen Metallsalze der Übergangsmetalle und Mischungen solcher Metallsalze in Frage. Da bekannt ist, dass Palladium mit einem Redoxpotential von 0,6 V (Pd(0)/Pd(II)) bereits in der Lage ist, Methan zu aktivieren, muss mindestens ein Übergangsmetall mit einem Redoxpaar mit einem Redoxpotential von größer 0,6 V, vorzugsweise größer 0,7 Volt, eingesetzt werden. Die besten Ergebnisse wurden mit Kobaltacetat und Kobaltnitrat erzielt. Das Normalpotential für das Redoxpaar Co (II) / (III) beträgt 0,92 Volt. Beim Einsatz von Mangan (III) - acetat konnte unter identischen Bedingungen eine geringere Ausbeute, aber noch immer eine deutlich katalytische Reaktion erreicht werden.

Nach Hollemann Wiberg, 81.-90. Auflage, 1976, S. 202 ff., beträgt das Normalpotential von Mn²⁺ nach Mn³⁺ in saurer Lösung +1,51 V. Das Normalpotential von Pd nach Pd²⁺ beträgt 0,987, und von CO²⁺ nach CO³⁺ 1,808.

Für die katalytische Oxidation von Methan sind insbesondere Salze von Palladium, Kobalt und Mangan bevorzugt. Unter diesen sind die Salze des Kobalts besonders geeignet.

Nach einer erfindungsgemäßen Ausführungsform wird als Übergangsmetallsalz Mangan(III)-acetat und/oder Mangannitrat verwendet.

Die Erfindung ist durch die nachstehenden Beispiele in nicht einschränkender Weise erläutert.

### Vergleichsbeispiel 1

### Katalyseversuche in reiner Trifluoressigsäure

Als Reaktionsmedium dienten 70 ml reiner Trifluoressigsäure, in welcher 1000 mg (etwa 3,8 Mol-% bezüglich Methan) Kobalt(II)acetat-Tetrahydrat gelöst wurden. Mit einem 2 : 1 Gasgemisch von Methan zu Sauerstoff (Vol.) wurde ein Anfangsdruck von 30 bar hergestellt; die Mischung wurde im Autoklaven auf 180°C erhitzt. Nach dem Abkühlen und Entspannen des Autoklaven wurde von der flüssigen Phase ein Gaschromatographie (GC) Spektrum aufgenommen. Darin konnte der Ester eindeutig identifiziert und die Esterausbeute zu etwa 17%, bezogen auf Methan, ermittelt werden. Gleiche Werte wurden beim Einsatz von Kobaltnitrat erzielt.

### Beispiel 1

### Katalyseversuche in Trifluoressigsäure/ Trifluoressigsäureanhydrid

Als Reaktionsmedium diente ein Gemisch aus 60 ml reiner Trifluoressigsäure und 10 ml Trifluoressigsäureanhydrid, in welchem 1000 mg (etwa 3,8 Mol-% bezüglich Methan) Kobalt(II)acetat-Tetrahydrat gelöst wurden. Mit einem 2 : 1-Gasgemisch von Methan zu Sauerstoff (Vol.) wurde ein Anfangsdruck von 30 bar hergestellt, und das Gemisch wurde im Autoklaven auf 180°C erhitzt. Nach 24 Stunden wurde bei 180°C ein Druckabfall von 67 bar auf 47 bar registriert, was einem Methanumsatz von fast 30% entspricht. Nach dem Abkühlen und Entspannen des Autoklaven wurde von der flüssigen Phase ein GC-Spektrum aufgenommen. Darin konnte der Ester eindeutig identifiziert und die Esterausbeute zu etwa 26%, bezogen auf Methan, ermittelt werden.

Dass die Vorgänge bei der vorgestellten Katalyse nicht so einfach liegen, unterstreicht die Beobachtung, dass nach den Reaktionen Niederschläge in den Produktlösungen auftreten. Eine Elementaranalyse hat ergeben, dass die ungefähre Zusammensetzung der von CoF₂ entspricht. Die Unlöslichkeit des gebildeten Kobaltfluorids in Trifluoressigsäure hat zur Folge, dass der Katalysator deaktiviert wird.

Bei deutlich verkürzten Reaktionszeiten von 14 bzw. 11 Stunden konnten die bisher erhaltenen Esterausbeuten im Bereich von 21 bis 26% ebenfalls erhalten werden. Wie bereits zuvor vermutet wurde, hat eine längere Reaktionszeit keinen weiteren Einfluss mehr auf die Esterausbeute. Die Ursachen sind auch hier die aufgetretenen Niederschläge von katalytisch inaktivem CoF₂.

Durch eine weitere Reduktion der Reaktionszeiten sollte geklärt werden, zu welchem Zeitpunkt die Methan-Aktivierung einsetzt und wann es zur Deaktivierung des Katalysators durch Bildung von unlöslichem Kobaltfluorid kommt.

### Kinetikmessungen mit dem Kobalt-System

Unter den zuvor angewendeten Bedingungen wurden eine Reihe von Katalysereaktionen durchgeführt, bei denen die Reaktionszeit sukzessive herabgesetzt wurde. Es sollte festgestellt werden, nach welcher Zeit die Maximalausbeute an Ester erreicht bzw. der Katalysator deaktiviert wird. Um eine Reaktion schon während der Aufheizphase des Autoklaven zu verhindern, wurde zunächst nur mit sehr kleinen Umdrehungszahlen gerührt, um einerseits den Wärmetransport zu gewährleisten und andererseits den Gaseintrag in die Lösung minimal zu halten. Nach dem Erreichen der Zieltemperatur wurde die Umdrehungszahl von etwa 400 min⁻¹ auf 1.500 min⁻¹ erhöht und ab diesem Zeitpunkt die Reaktionszeit gemessen. In der Versuchsreihe kamen jeweils 5 Mol-% Kobaltacetat zum Einsatz.

Nach einer Reaktionszeit von drei Stunden wurde bereits eine Esterausbeute von mehr als 26% bezogen auf Methan registriert. Nach zwei Stunden betrug der Umsatz fast 20%, während nach einer Stunde lediglich Spuren des Esters und nach nur 30 min. kein Ester nachzuweisen war.

Während der ersten Stunde der Reaktionszeit bei 280°C wird das gewünschte Produkt demnach noch nicht gebildet, allerdings wird nach dieser Zeit auch kein Niederschlag von Kobaltfluorid und damit eine Deaktivierung des Katalysators beobachtet.

Die Ursache für diese Induktionsperiode ist noch nicht bekannt.

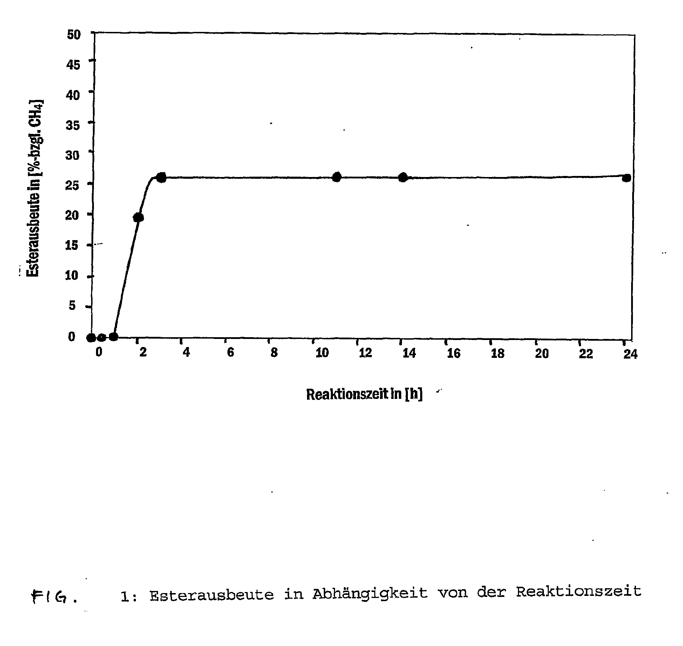

Die Verhältnisse sind in Abbildung 1 angegeben.

### Beispiel 2

Die Arbeitsweise von Beispiel 1 wurde mit der Abweichung wiederholt, dass statt Kobalt (III)-acetat-Tetrahydrat 3,8 Mol-% Mangan(III)-acetat verwendet wurden. Beim Einsatz von Mangan(III)-acetat konnte unter den im Vergleichsbeispiel 1 aufgeführten, identischen Bedingungen immerhin eine Esterausbeute von etwa 15%, bezogen auf Methan, ermittelt werden.

### Beispiel 3

### Katalyseversuche in Trifluoressigsäure/Essigsäure-Gemischen

Mit einem Gemisch aus 40 ml Trifluoressigsäure und 30 ml Essigsäure (Stoffmengenverhältnis 1 : 1) wurden Katalyseversuche wie bisher bei 180°C und einem Anfangsdruck von etwa 30 bar (Methan/Sauerstoff 2 : 1) durchgeführt. Die Reaktionszeiten lagen jeweils bei 20 Stunden, in deren Verlauf jeweils ein deutlicher Druckverlust beobachtet wurde. Nach dem Aufarbeiten der Ansätze konnten keine Niederschläge von Kobaltfluoriden beobachtet werden.

Die GC-Analysen der flüssigen Phasen ergaben, dass sich im Verlauf der Reaktion nicht wie erhofft nur der Trifluoressigsäuremethylester, sondern auch der Essigsäuremethylester gebildet hatte. Durch den Vergleich mit einer Referenzprobe konnten die Peaks eindeutig identifiziert werden. Die Ausbeuten bezüglich Methan lagen für den Trifluoressigsäuremethylester im Bereich von 6,5 bis 12,5% und für den Essigsäuremethylester bei 11,3 bis 15,5%.

Ein Vergleich der Peakflächen-Verhältnisse zwischen Trifluoressigsäure und Essigsäure vor und nach der Reaktion wurde angestellt. Vor der Reaktion betrug dass Verhältnis Trifluoressigsäure/Essigsäure 0,2312 und nach der Reaktion 0,2777. Ein Grund für die beobachtete Zunahme des Verhältnisses ist darin zu sehen, dass mehr Essigsäuremethylester als Trifluoressigsäuremethylester gebildet wird.

## Patentansprüche

1. Verfahren zur Oxidation von Methan zu Methanol über Methanolester, wobei die Oxidation des Methans mit Sauerstoff oder einem sauerstoffhaltigen Gas in Gegenwart eines oder mehrerer Salze aus der Gruppe der Übergangsmetalle, die ein Redoxpaar mit einem Normalpotential > 0,6 Volt bilden, als Katalysator in Trifluoressigsäure durchgeführt und die Ester zu Methanol hydrolysiert werden, **dadurch gekennzeichnet, dass** man die Oxidation in einer Mischung aus Trifluoressigsäure und einem Carbonsäureanhydrid und/oder in einer Mischung aus Trifluoressigsäure und einer anderen, nicht fluorierten Carbonsäure bei Temperaturen von mehr als 80°C durchführt.

2. Verfahren nach Anspruch 1, **dadurch gekennzeichnet, dass** man als Carbonsäureanhydrid, das im Gemisch mit Trifluoressigsäure eingesetzt wird, Trifluoressigsäureanhydrid verwendet.

3. Verfahren nach Anspruch 1 oder 2, **dadurch gekennzeichnet, dass** man das Gewichtsverhältnis zwischen Trifluoressigsäure und Carbonsäureanhydrid auf 99:1 bis 1:99, vorzugsweise auf 99:1 bis 20:80, insbesondere auf 70:30 bis 30:70 einstellt.

4. Verfahren nach Anspruch 1, **dadurch gekennzeichnet, dass** man als nicht fluorierte Carbonsäure, die im Gemisch mit Trifluoressigsäure eingesetzt wird, Essigsäure verwendet.

5. Verfahren nach Anspruch 4, **dadurch gekennzeichnet, dass** man das Gewichtsverhältnis zwischen Trifluoressigsäure und nicht fluorierter Carbonsäure auf 99:1 bis 1:99, vorzugsweise auf 99:1 bis 5:95, besonders bevorzugt auf 99:1 bis 10:90, insbesondere auf 30:70 bis 70:30, einstellt.

6. Verfahren nach einem der Ansprüche 1 bis 5, **dadurch gekennzeichnet, dass** man als Übergangsmetallsalz ein Salz des Kobalts verwendet.

7. Verfahren nach einem der Anspruch 6, **dadurch gekennzeichnet, dass** man als Übergangsmetallsalz Kobaltacetat und/oder Kobaltnitrat verwendet.

8. Verfahren nach einem der Ansprüche 1 bis 5, **dadurch gekennzeichnet, dass** man als Übergangsmetallsalz ein Salz des Mangans verwendet.

9. Verfahren nach Anspruch 8, **dadurch gekennzeichnet, dass** man als Übergangsmetallsalz Mangan(III)-acetat und/oder Mangannitrat verwendet.

## Claims

1. A method for the oxidation of methane to form methanol via methanol esters, wherein the oxidation of methane with oxygen or a gas containing oxygen is carried out in the presence of one or more salts from the group of the transition metals which form a redox pair with a normal potential of > 0.6 volts as a catalyst in trifluoro acetic acid and the esters are hydrolysed to form methanol, **characterised in that** the oxidation is carried out in a mixture of trifluoro acetic acid and a carboxylic acid anhydride and/or in a mixture of trifluoro acetic acid and another non-fluorinated carboxylic acid at temperatures of more than 80°C.

2. A method according to Claim 1, **characterised in that** trifluoro acetic acid anhydride is used as carboxylic acid anhydride which is used in mixture with trifluoro acetic acid.

3. A method according to Claim 1 or 2, **characterised in that** the weight ratio between trifluoro acetic acid and carboxylic acid anhydride is adjusted to 99:1 to 1:99, preferably to 99:1 to 20:80, in particular to 70:30 to 30:70.

4. A method according to Claim 1, **characterised in that** acetic acid is used as non-fluorinated carboxylic acid which is used in mixture with trifluoro acetic acid.

5. A method according to Claim 4, **characterised in that** the weight ratio between trifluoro acetic acid and non-fluorinated carboxylic acid is adjusted to 99:1 to 1:99, preferably to 99:1 to 5:95, especially preferably to 99:1 to 10:90, in particular to 30:70 to 70:30.

6. A method according to any one of Claims 1 to 5, **characterised in that** a salt of cobalt is used as transition metal salt.

7. A method according to [sic] Claim 6, **characterised in that** cobalt acetate and/or cobalt nitrate is used as transition metal salt.

8. A method according to Claims 1 to 5, **characterised in that** a salt of manganese is used as transition metal salt.

9. A method according to Claim 8, **characterised in that** manganese (III) acetate and/or manganese nitrate is used as transition metal salt.

## Revendications

1. Procédé pour l'oxydation du méthane en méthanol, par l'intermédiaire d'un ester de méthanol, dans lequel l'oxydation du méthane avec de l'oxygène ou avec un gaz contenant de l'oxygène est effectuée dans de l'acide trifluoracétique en présence, comme catalyseur, d'un ou de plusieurs sels du groupe des métaux de transition qui forment une paire redox avec un potentiel normal > 0,6 volt, et dans lequel les esters sont hydrolysés en méthanol, **caractérisé en ce que** l'on effectue l'oxydation dans un mélange d'acide trifluoracétique et d'un anhydride d'acide carboxylique et/ou dans un mélange d'acide trifluoracétique et d'un autre acide carboxylique non fluoré à des températures de plus de 80 °C

2. Procédé selon la revendication 1, **caractérisé en ce que**, comme anhydride d'acide carboxylique que l'on met en oeuvre dans un mélange avec l'acide trifluoracétique, on utilise de l'anhydride trifluoracétique.

3. Procédé selon la revendication 1 ou 2, **caractérisé en ce que** l'on règle le rapport pondéral entre l'acide trifluoracétique et l'anhydride d'acide carboxylique entre 99 : 1 et 1 : 99, de préférence entre 99 : 1 et 20 : 80, en particulier entre 70 : 30 et 30 : 70.

4. Procédé selon la revendication 1, **caractérisé en ce que**, comme acide carboxylique non fluoré que l'on met en oeuvre dans un mélange avec l'acide trifluoracétique, on utilise de l'acide acétique.

5. Procédé selon la revendication 4, **caractérisé en ce que** l'on règle le rapport pondéral entre l'acide trifluoracétique et l'acide carboxylique non fluoré entre 99 : 1 et 1 : 99, de préférence entre 99 : 1 et 5 : 95, de façon particulièrement préférée entre 99 : 1 et 10 : 90, en particulier entre 70 : 30 et 30 : 70.

6. Procédé selon l'une des revendications 1 à 5, **caractérisé en ce que**, comme sel de métal de transition, on utilise un sel de cobalt.

7. Procédé selon la revendication 6, **caractérisé en ce que**, comme sel de métal de transition, on utilise de l'acétate de cobalt et/ou du nitrate de cobalt.

8. Procédé selon l'une des revendications 1 à 5, **caractérisé en ce que**, comme sel de métal de transition, on utilise un sel de manganèse.

9. Procédé selon la revendication 8, **caractérisé en ce que**, comme sel de métal de transition, on utilise de l'acétate de manganèse(III) et/ou du nitrate de manganèse.
